# EUROPEAN PATENT SPECIFICATION

(11) **EP 1 004 321 B1**
(45) Date of publication and mention of the grant of the patent: **26.02.2003**
(21) Application number: 98937814.6
(22) Date of filing: 13.08.1998
(51) Int. Cl.: A61K 51/00, A61K 51/04, A61K 51/12

(54) **STABLE RADIOACTIVE MEDECINE**
STABILE RADIOAKTIVE MEDIKAMENTE
MEDICAMENT RADIOACTIF STABLE

(30) Priority: 14.08.1997 JP 23186397
(43) Date of publication of application: 31.05.2000
(73) Proprietor: DAIICHI RADIOISOTOPE LABORATORIES, LTD., Tokyo 104-0031 (JP)
(72) Inventor: HATSUSHIBA, Kiyonori, Ichihara-shi, Chiba-ken 290-0062 (JP); KAWAKAMI, Takeshi, Togane-shi, Chiba-ken 283-0066 (JP); INOUE, Minoru, Sanb-gun, Chiba-ken 289-1345 (JP); ISHIDA, Satoshi, Togane-shi, Chiba-ken 283-0005 (JP)
(74) Representative: HOFFMANN - EITLE
(86) International application number: JP9803615
(87) International publication number: WO99008709

(56) References cited:
- JP-A- 1 160 923
- JP-A- 56 097 871
- US-A- 4 826 966
- MALLOL J., "Optimal Preservation of Radiolabeled Thyroxine Against Deiodination by Storage in Phosphate Buffer Protein", REVUE IRE TIJDSCHRIFT, 1985, Vol. 9, No. 4, pp. 28-30, XP002917004

## Description

The present invention relates to a stable radioactive medicine. More particularly, the present invention relates to a stable radioactive medicine which can maintain a certain level of radioactive chemical purity, while preventing a radioactive iodine-labeled compound contained therein from being deiodinated.

### BACKGROUND ART

At present, a drug composition which contains a radioactive iodine-labeled compound is being supplied as one radioactive medicine. This radioactive medicine has a radioactive iodine incorporated in the basic skeleton of a specific compound. The radioactive iodine is accumulated in a specific internal organ utilizing the specific accumulative characteristics of the compound for said specific internal organ or specific diseases. Radiation emitted from the accumulated radioactive iodine is measured from outside the body to diagnose the presence or absence of diseases or their functions, or to diagnose functions of internal organs.

However, because the radioactive medicine using this radioactive iodine is easily deiodinated if left as is, its radiochemical purity decreases over time. Even if the medicine has no problem with radiochemical purity immediately after preparation, the radiochemical purity decreases with passage of time, leaving only an insufficient radiochemical purity when the medicine is administered to a patient.

If such a radioactive medicine with decreased radiochemical purity is administered in vivo, radioactive iodine which is produced by deiodination and independently present in vivo may accumulate specifically in the thyroid gland as a natural result of metabolism. The thyroid gland may be unnecessarily exposed to radiation which is emitted from the radioactive iodine accumulated in the thyroid gland. When measuring distribution of the radioactive medicine throughout the whole body, the radioactive iodine accumulated in the thyroid gland at a high concentration may affect the site which should be diagnosed.

A conventional method for avoiding such a problem has been to administer a non-radioactive iodine preparation in advance to preclude adsorption of extra iodine in the thyroid gland, thereby reducing incorporation of radioactive iodine in the thyroid gland. There will be no problem if the thyroid gland is previously blocked. However, such blocking of the thyroid gland is sometimes insufficient in actual clinical practice. The problem is particularly serious in the case of medicines that are used for infant diseases for which total body photographing is more frequently adopted.

Therefore, there has been a strong desire for the development of a technology which can maintain radioactive iodine in a stable manner and prevent decrease in the radioactive chemical purity in radioactive medicines containing radioactive iodine.

### DISCLOSURE OF THE INVENTION

The inventors of the present invention have conducted extensive studies on measures for preventing a deiodination reaction which is a cause of instability in radioactive medicines containing radioactive iodine by the addition of some compound. Because the compounds will be intravenously administered, conditions established for the study included not only possession of deiodination activity, but also low toxicity to humans, comparatively excellent solubility in water, and capability of exhibiting sufficient deiodination in a small dose. As a result of investigation of a wide variety of compounds, certain compounds have been found to satisfy these conditions.

Specifically, an object of the present invention is to provide a stable radioactive medicine which comprises a radioactive iodine labeled compound and a deiodination inhibitor which is selected from the group consisting of α-thioglycerol, sodium hydrogensulfite, sodium pyrosulfite, cysteine hydrochloride, sodium edetate, sodium thioglycollate, and butylhydroxyanisole.

### BRIEF DESCRIPTION OF THE DRAWINGS

Fig. 1 is a drawing showing the decrease in radiochemical purity when a radioactive medicine containing 9MPA labeled with radioactive iodine 123 and α-thioglycerol was stored at 25°C.

Fig. 2 is a drawing showing the decrease in radiochemical purity when the same radioactive medicine was stored at 37°C.

### BEST MODE FOR CARRYING OUT THE INVENTION

There are no specific limitations to the radioactive iodine-labeled compound used in the stable radioactive medicine of the present invention inasmuch as such a compound is an organic compound having a radioactive iodine bonded to the skeleton as a labeling compound. Given as specific examples are a heart imaging agent such as radioactive iodine 123-labeled 3-iodo benzyl guanidine, radioactive iodine 131-labeled 3-iodo benzyl guanidine, or radioactive iodine 123-labeled 15-(4-iodophenyl)-9-methylpentadecanoic acid, cerebral blood flow imaging agent such as radioactive iodine 123-labeled N-isopropyl-p-iodo amphetamine, and the like.

Given as specific compounds which can be used as a deiodination inhibitor in the stable radioactive medicine of the present invention are compounds which are selected from the group consisting of α-thioglycerol, sodium hydrogensulfite, sodium pyrosulfite, cysteine hydrochloride, sodium edetate, sodium thioglycollate, and butylhydroxyanisole. These compounds can exhibit a sufficient deiodination effect with the addition of a certain amount and, at the same time, exhibit only a low toxicity to humans and are comparatively easily dissolved in aqueous solutions.

Maximum allowable doses for intravenous administration of these compounds are as follows: 25 mg for α-thioglycerol, 800 mg for sodium hydrogensulfite, 40 mg for sodium pyrosulfite, 25 mg for cysteine hydrochloride, 16 mg for sodium edetate, 20 mg for sodium thioglycollate, and 75 µg for butylhydroxyanisole. All these maximum allowable doses are greater than the amount required for these compounds to exhibit sufficient effect.

On the other hand, the amount of these deiodination inhibitors to be added to specific radioactive iodine-labeled compounds in the radioactive medicine of the present invention is determined according to the maximum intravenous dose described in Pharmaceutical Additives Dictionary (edited by The Japan Drug Additives Institute, recommended by the Ministry of Health and Welfare, Pharmaceutical Affairs Bureau, published by Pharmaceutical Daily Report Company (1995)).

Such an amount of deiodination inhibitor varies according to the type of radioactive iodine-labeled compound to be added. For example, in the case of injection of 3-iodo benzyl guanidine labeled with radioactive iodine 123, the amount of deiodination inhibitors added for 1.0 ml of such an injection is as follows: α-thioglycerol in the range from 0.5-25 mg, sodium hydrogensulfite in the range from 5.2-800 mg, sodium pyrosulfite in the range from 5.7-40 mg, cysteine hydrochloride in the range from 3.5-25 mg, sodium edetate in the range from 9.3-16 mg, sodium thioglycollate in the range from 17-20 mg, and butylhydroxyanisole in the range from 22.5-75 µg.

The most preferable amount for 1.0 ml of the injection of 3-iodo benzyl guanidine labeled with radioactive iodine 123 is about 2.5 mg of α-thioglycerol, about 26 mg of sodium hydrogensulfite, about 28 mg of sodium pyrosulfite, about 10 mg of cysteine hydrochloride, about 10 mg of sodium edetate, about 17 mg of sodium thioglycollate, or about 55 µg of butylhydroxyanisole.

The radioactive medicine of the present invention can be prepared by adding a deiodination inhibitor to a radioactive iodine-labeled compound in accordance with any one of the following methods.

A first method comprises preparing a radioactive medicine containing a radioactive iodine-labeled compound according to a conventional method, adding a required amount of liquid or powdered stabilizer, sterilizing the mixture using filtration or the like means, and subdividing the resulting medicine.

The use of an anion exchange column to eliminate unreacted radioactive iodine from the mixture is indispensable in the preparation of radioactive medicine. In a second method for preparing such a radioactive medicine, a stabilizer is previously added to an eluate, diluting solution, or dissolving solution used in this elimination process using an anion exchange column, so that a prescribed amount of the stabilizer may be included in the radioactive medicine.

There are various hypotheses for the reasons for chemical and physical characteristics of the above-described deiodination inhibitor, particularly, for the reasons for the effect of deiodination in the radioactive medicine of the present invention. At the present time, however, no definitive reasons have been found.

One of the hypotheses is based on the preposition that deiodination occurs in a radioactive medicine supplied in an aqueous form due to production of a peroxide by continuous irradiation of radioactive rays during preparation and thereafter. The peroxide attacks the iodine atom and releases the iodine atom from the bonding site. The deiodination inhibitor of the present invention is thought to neutralize the peroxide by the antioxidative action and to prevent deiodination.

### EXAMPLES

The present invention will be described in more detail by way of Examples, Reference Examples, and Test Examples.

### Reference Example 1

### Preparation of 3-iodo benzyl guanidine labeled with radioactive iodine 123

3-iodo benzyl guanidine labeled with radioactive iodine 123 was prepared according to the method of Example 1 of Japanese Patent No. 2546697. A 50 ml eggplant-type flask with a small stirrer made from Teflon placed therein was charged with 500 µl of 3-iodo benzyl guanidine solution in methanol at a concentration of 1 mg/ml prepared using 50% methanol, 20 mg of ammonium sulfate (manufactured by Kishida Chemical Co., Ltd.), 10 µl of an aqueous solution of copper sulfate (manufactured by Kishida Chemical Co., Ltd.) prepared using water for injection at a concentration of 5 mg/ml, and 500 µl of water for injection.

Then, 200 µl of 0.1 M sodium hydroxide solution which contains radioactive iodine 123 in the form of sodium iodide with a radioactivity capacity of 25 GBq per 1 ml (manufactured by Daiichi Radioisotope Laboratories, Ltd.) was added. This eggplant-type flask was embedded in a heating block which was heated at 140-150°C. The solution was stirred using the stirrer for 20 minutes while slightly reducing the pressure using a pressure reducing pump, thereby carrying out both the reaction and concentration to dryness. After stopping the stirrer, the reaction was continued for a further 40 minutes under the same conditions.

The reaction product was dissolved in 2 ml of 5 mM acetic acid buffer (pH 4.5) and added to a column (5 ml Thermo syringe) packed with an anion exchange resin (DE52 made by Wattman Co.) up to 4 ml and equilibrated with 5 mM acetic acid buffer (pH 4.5). The initial eluate was discarded. 4 ml of eluate obtained by successive addition of 4 ml of 5 mM acetic acid buffer (pH 4.5) was collected. The radioactivity of this 4 ml of eluate was measured using a radioisotope calibrator (CRC-12R, manufactured by Capintec). The eluate was diluted with 5 mM acetic acid buffer (pH 4.5) so that the radioactivity of 1 ml of the solution would be 148 MBq/ml at noon of the next day (the calibration date).

Then, the solution was adjusted to give the final preparation of 50 mM acetic acid buffer (pH 4.5, containing 0.7% sodium chloride) using 95 mM acetic acid buffer (pH 4.5, containing 1.4% sodium chloride), sterilized by filtration through a 0.2 µm filter (Mylex GS manufactured by Millipore, Ltd.). 1 ml of the resulting preparation was dispensed into each of a number of 5 ml glass bottles.

### Example 1

### Preparation of a solution of α-thioglycerol (1-thioglycerol) in 3-iodo benzyl guanidine labeled with radioactive iodine 123 (1)

40 µl (47.5 mg), 20 µl (23.7 mg), 10 µl (11.9 mg), or 5 µl (5.93 mg) of 95% α-thioglycerol (manufactured by Kanto Chemical Co., Ltd.) was added to 1 ml of 3-iodo benzyl guanidine labeled with radioactive iodine 123 which was prepared in the Reference Example 1. The mixture was stirred sufficiently. After stirring, the container containing the radioactive medicine was capped with a rubber stopper. The product was stored at room temperature.

### Example 2

### Addition of sodium hydrogensulfite to a solution of 3-iodo benzyl guanidine labeled with radioactive iodine 123

A 52 mg/ml solution of sodium hydrogensulfite (manufactured by Kishida Chemical Co., Ltd.) in 50 mM acetic acid buffer (pH 4.5, containing 0.7% sodium chloride) was prepared. 100 µl (5.2 mg), 300 µl (15.6 mg), or 500 µl (26.0 mg) of this solution was added to the 3-iodo benzyl guanidine solution labeled with radioactive iodine 123 which was prepared in Reference Example 1. The mixture was stirred sufficiently and the container containing the same was capped with a rubber stopper, thus obtaining the radioactive medicine. The product was stored at room temperature.

### Example 3

### Addition of sodium pyrosulfite to a solution of 3-iodo benzyl guanidine labeled with radioactive iodine 123

A 57 mg/ml solution of sodium pyrosulfite (manufactured by Kanto Chemical Co., Ltd.) in 50 mM acetic acid buffer (pH 4.5, containing 0.7% sodium chloride) was prepared. 100 µl (5.7 mg), 300 µl (17.1 mg), or 500 µl (28.5 mg) of this solution was added to the 3-iodo benzyl guanidine solution labeled with radioactive iodine 123 which was prepared in Reference Example 1. The mixture was stirred sufficiently and the container containing the same was capped with a rubber stopper, thus obtaining the radioactive medicine. The product was stored at room temperature.

### Example 4

### Addition of cysteine hydrochloride to a solution of 3-iodo benzyl guanidine labeled with radioactive iodine 123

A 35.1 mg/ml solution of cysteine hydrochloride (manufactured by Kishida Chemical Co., Ltd.) in 50 mM acetic acid buffer (pH 4.5, containing 0.7% sodium chloride) was prepared. 100 µl (3.5 mg), 300 µl (10.5 mg), or 500 µl (17.1 mg) of this solution was added to the 3-iodo benzyl guanidine solution labeled with radioactive iodine 123 which was prepared in Reference Example 1. The mixture was stirred sufficiently and the container containing the same was capped with a rubber stopper, thus obtaining the radioactive medicine. The product was stored at room temperature.

### Example 5

### Addition of sodium edetate to a solution of 3-iodo benzyl guanidine labeled with radioactive iodine 123

A 18.6 mg/ml solution of sodium edetate (manufactured by Junsei Chemical Co., Ltd.) in 50 mM acetic acid buffer (pH 4.5, containing 0.7% sodium chloride) was prepared. 500 µl (9.3 mg) of this solution was added to the 3-iodo benzyl guanidine solution labeled with radioactive iodine 123 which was prepared in Reference Example 1. The mixture was stirred sufficiently and the container containing the same was capped with a rubber stopper, thus obtaining the radioactive medicine. The product was stored at room temperature.

### Example 6

### Addition of sodium thioglycollate to a solution of 3-iodo benzyl guanidine labeled with radioactive iodine 123

A 34.2 mg/ml solution of sodium thioglycollate (manufactured by Kishida Chemical Co., Ltd.) in 50 mM acetic acid buffer (pH 4.5, containing 0.7% sodium chloride) was prepared. 500 µl (17.1 mg) of this solution was added to the 3-iodo benzyl guanidine solution labeled with radioactive iodine 123 which was prepared in Reference Example 1. The mixture was stirred sufficiently and the container containing the same was capped with a rubber stopper in this condition, thus obtaining the radioactive medicine. The product was stored at room temperature.

### Example 7

### Addition of butylhydroxyanisole to a solution of 3-iodo benzyl guanidine labeled with radioactive iodine 123

A 7.5 mg/ml solution of sodium butylhydroxyanisole (manufactured by Kishida Chemical Co., Ltd.) in ethanol (manufactured by Kishida Chemical Co., Ltd.) was prepared. 3 µl (22.5 mg), 7 µl (52.5 mg), or 10 µl (75.0 mg) of this solution was added to the 3-iodo benzyl guanidine solution labeled with radioactive iodine 123 which was prepared in Reference Example 1. The mixture was stirred sufficiently and the container containing the same was capped with a rubber stopper in this condition, thus obtaining the radioactive medicine. The product was stored at room temperature.

### Example 8

### Addition of a solution of α-thioglycerol in 3-iodo benzyl guanidine labeled with radioactive iodine 123 (2)

α-thioglycerol was added to and dissolved in all acetic acid buffer solutions used for the dissolution, purification, and dilution steps in the preparation of radioactive iodine 123-labeled 3-iodo benzyl guanidine so that the α-thioglycerol concentration became 6 mg/ml.

3-iodo benzyl guanidine labeled with radioactive iodine 123 was prepared in the same manner as in Reference Example 1 using the buffer solution which contains this α-thioglycerol. In addition, the anion exchange column equilibrated with the buffer solution which contains this α-thioglycerol was used for purification. The labeled material was adjusted so that the radioactivity became 74 MBq/ml at noon of the next day (the calibration date) and 1 ml aliquots of the solution were dispensed in 5 ml glass bottles. The glass bottles were capped with a rubber stopper to obtain a radioactive medicine containing 6.25 mg of α-thioglycerol. The product was stored at room temperature and in an incubator at 37°C.

### Example 9

### Measurement of radiochemical purity

The radiochemical purity of the radioactive medicines of the present invention prepared in Examples 1-8 was measured 24 hours and 48 hours after preparation. Solutions before the addition of the deiodination inhibitors in Examples 1-8 were used as controls, provided, however, no control solution for Example 8 could be obtained because of the experimental procedure. The results are shown hereinafter.

### The method for measurement of radiochemical purity

A reverse phase thin layer chromatograph (manufactured by Wattman Co., Glass mount KC18F, 200×200 mm) was cut into 200 mm × 10 mm sections. Two lines parallel to the short side were drawn using a pencil at 20 mm and 120 mm from the bottom. 2 µl of each solution of the radioactive medicines prepared in Examples 1-8 was dropped around the center of the line drawn at 20 mm from the bottom and developed using 80% methanol (methanol:water = 4:1) as a solvent before drying the dropped spot. The development was deemed to have been completed when the developing solvent reached the line which was drawn at 120 mm from the bottom. After air drying, the surface of the thin layer was coated with cellophane tape to measure the distribution of radioactive iodine 123 on the thin layer using a radio thin layer chromatography analyzer (Radiochromanizer manufactured by Aloka Co., Ltd.).

The transfer rate of radioactive iodine 123 alone in this reverse phase thin layer chromatography was about 0.9, whereas the transfer rate of 3-iodo benzyl guanidine bonded with radioactive iodine 123 was about 0, indicating that it is possible to quantitatively measure the radioactive iodine 123 which is present unreacted in the solution or released from 3-iodo benzyl guanidine.

### Results

The radiochemical purity (hereinafter referred to simply as "purity") and the rate of decrease of the radioactive medicines of the present invention and controls at 24 hours and 48 hours after preparation, as compared with the purity of the product immediately after the preparation in Reference Example 1, are shown in Tables 1-8. Tables 1-7 respectively show the results of α-thioglycerol, sodium hydrogensulfite, sodium pyrosulfite, cysteine hydrochloride, sodium edetate, sodium thioglycollate, and butylhydroxyanisole. Table 8 shows the results of measurement of a solution of radioactive iodine 123-labeled 3-iodo benzyl guanidine containing α-thioglycerol.

**Table 1**

| The amount of α-thioglycerol | After 24 hour storage | | After 48 hour storage | |
|---|---|---|---|---|
| | Purity (%) | Rate of decrease*(%) | Purity (%) | Rate of decrease*(%) |
| 5 µl (6.25 mg) | 99.6 | 0.1 | 99.7 | 0 |
| 10 µl (12.5 mg) | 99.7 | 0 | 99.7 | 0 |
| 20 µl (25.0 mg) | 99.8 | 0 | 99.8 | 0 |
| 40 µl (50.0 mg) | 99.8 | 0 | 99.9 | 0 |
| Control | 98.4 | 1.3 | 97.5 | 2.2 |

| | | | | |
|---|---|---|---|---|
| * The rate of decrease from the purity of 99.7% immediately after preparation | | | | |

**Table 2**

| The amount of sodium hydrogensulfite | After 24 hour storage | | After 48 hour storage | |
|---|---|---|---|---|
| | Purity (%) | Rate of decrease* (%) | Purity (%) | Rate of decrease* (%) |
| 100 µl (5.20 mg) | 96.4 | 2.3 | 96.4 | 2.3 |
| 300 µl (15.6 mg) | 97.0 | 1.7 | 97.5 | 1.2 |
| 500 µl (26.0 mg) | 97.3 | 1.4 | 97.8 | 0.9 |
| Control | 95.9 | 2.8 | 95.0 | 3.7 |

| | | | | |
|---|---|---|---|---|
| * The rate of decrease from the purity of 98.7% immediately after preparation | | | | |

**Table 3**

| The amount of sodium pyrosulfite | After 24 hour storage | | After 48 hour storage | |
|---|---|---|---|---|
| | Purity (%) | Rate of decrease* (%) | Purity (%) | Rate of decrease* (%) |
| 100 µl (5.70 mg) | 97.0 | 1.7 | 96.9 | 1.8 |
| 300 µl (17.1 mg) | 97.0 | 1.7 | 97.0 | 1.7 |
| 500 µl (28.5 mg) | 97.5 | 1.2 | 97.1 | 1.6 |
| Control | 95.9 | 2.8 | 95.0 | 3.7 |

| | | | | |
|---|---|---|---|---|
| * The rate of decrease from the purity of 98.7% immediately after preparation | | | | |

**Table 4**

| The amount of cysteine hydrochloride | After 24 hour storage | | After 48 hour storage | |
|---|---|---|---|---|
| | Purity (%) | Rate of decrease* (%) | Purity (%) | Rate of decrease* (%) |
| 100 µl (3.51 mg) | 97.9 | 0.8 | 97.1 | 1.6 |
| 300 µl (10.5 mg) | 98.0 | 0.7 | 97.5 | 1.2 |
| 500 µl (17.6 mg) | 98.1 | 0.6 | 97.6 | 1.1 |
| Control | 95.9 | 2.8 | 95.0 | 3.7 |

| | | | | |
|---|---|---|---|---|
| * The rate of decrease from the purity of 98.7% immediately after preparation | | | | |

**Table 5**

| The amount of sodium edetate | After 24 hour storage | | After 48 hour storage | |
|---|---|---|---|---|
| | Purity (%) | Rate of decrease* (%) | Purity (%) | Rate of decrease* (%) |
| 500 µl (9.30 mg) | 97.9 | 1.1 | 96.3 | 2.7 |
| Control | 97.3 | 1.7 | 95.2 | 3.8 |

| | | | | |
|---|---|---|---|---|
| * The rate of decrease from the purity of 99.0% immediately after preparation | | | | |

**Table 6**

| The amount of sodium thioglycollate | After 24 hour storage | | After 48 hour storage | |
|---|---|---|---|---|
| | Purity (%) | Rate of decrease*(%) | Purity (%) | Rate of decrease*(%) |
| 500 µl (17.1 mg) | 98.0 | 1.0 | 97.3 | 1.7 |
| Control | 97.3 | 1.7 | 95.2 | 3.8 |

| | | | | |
|---|---|---|---|---|
| * The rate of decrease from the purity of 99.0% immediately after preparation | | | | |

**Table 7**

| The amount of butylhydroxyanisole | After 24 hour storage | | After 48 hour storage | |
|---|---|---|---|---|
| | Purity (%) | Rate of decrease* (%) | Purity (%) | Rate of decrease* (%) |
| 3 µl (22.5 mg) | 97.2 | 2.0 | 96.8 | 2.4 |
| 7 µl (52.5 mg) | 97.4 | 1.8 | 97.2 | 2.0 |
| 10 µl (75.0 mg) | 97.5 | 1.7 | 97.4 | 1.8 |
| Control | 96.5 | 2.7 | 95.2 | 4.0 |

| | | | | |
|---|---|---|---|---|
| * The rate of decrease from the purity of 99.2% immediately after preparation | | | | |

**Table 8**

| Storage conditions | After 24 hour storage | | After 48 hour storage | |
|---|---|---|---|---|
| | Purity (%) | Rate of decrease* (%) | Purity (%) | Rate of decrease* (%) |
| Room Temp. (1) | 99.1 | 0 | 99.0 | 0.1 |
| Room Temp. (2) | 99.0 | 0.1 | 98.9 | 0.2 |
| 37°C (1) | 98.9 | 0.2 | 98.9 | 0.2 |
| 37°C (2) | 99.0 | 0.1 | 98.9 | 0.2 |

| | | | | |
|---|---|---|---|---|
| * The rate of decrease from the purity of 99.1% immediately after preparation | | | | |

### Example 10

### Preparation of a radioactive medicine and confirmation of radioactive iodine-labeled compound distribution in healthy rats using the medicine

### (1) Preparation of a radioactive medicine for confirmation of in vivo distribution

A solution of 3-iodo benzyl guanidine labeled with radioactive iodine 123 was prepared according to the method of Reference Example 1. 6 mg of α-thioglycerol, 25 mg of cysteine hydrochloride, or 20 mg of sodium thioglycollate was added to 1 ml of this solution. 2 ml aliquots of each solution thus prepared were added to 5 ml glass bottles. The bottles were capped with rubber stoppers and stored at room temperature.

After 20 hours, a solution of α-thioglycerol in radioactive iodine 123-labeled 3-iodo benzyl guanidine was diluted 50-fold with an α-thioglycerol solution which was adjusted to a concentration of 0.24 mg/ml using a physiological saline solution (manufactured by Hikari Pharmaceutical Co.). This solution was used as test radioactive medicine 1. Test radioactive medicine 2 was prepared in the same manner by diluting the solution of cysteine hydrochloride in radioactive iodine 123-labeled 3-iodo benzyl guanidine 50-fold with a cysteine hydrochloride solution which was adjusted to a concentration of 1.0 mg/ml using the physiological saline solution. Test radioactive medicine 3 was prepared in the same manner by diluting the solution of sodium thioglycollate in radioactive iodine 123-labeled 3-iodo benzyl guanidine 50-fold with a sodium thioglycollate solution which was adjusted to a concentration of 0.8 mg/ml using the physiological saline solution. The three test radioactive medicines were used in the next test as solutions for confirmation of in vivo distribution using healthy rats. In the last procedure of this test, each deiodination inhibitor to be administered to one rat was diluted to ten-fold of the concentration to be actually administered to humans, assuming that the body weights of a human adult and a rat are respectively 60 kg and 0.2 kg.

### (2) Confirmation of radioactive iodine 123-labeled 3-iodo benzyl guanidine distribution in healthy rats

A test for accumulation in the hearts of healthy rats was carried out using the three test radioactive medicines prepared in (1) above and radioactive medicines to which no deiodination inhibitor was added (control medicines) as test solutions. Three rats were used for each point in the test, wherein the cuticle of left hind legs of the rats was cut to inject 740 kBq/300 µl of each test solution from the exposed femoral vein. The carotid artery of the rats was cut 30 minutes and 240 minutes after the injection.

After bleeding, the chest was opened to extract the heart, liver, lungs, spleen, thyroid gland, adrenal body, and kidneys. The blood sample collected during the bleeding was used for the test. The radioactivities of the collected blood samples and various internal organs were measured using a gamma counter (Desktop-type MINAXI gamma counter, Autogamma 5530, manufactured by Packard Co.) The results at 30 minutes and 240 minutes after administration are shown in Tables 9 and 10, respectively.

**Table 9**

| % Dose/g | | | | |
|---|---|---|---|---|
| | α-Thioglycerol | Cysteine hydrochloride | Sodium thioglycollate | Control |
| Blood | 0.1946 | 0.1755 | 0.1889 | 0.2113 |
| Heart | 4.6801 | 4.7764 | 5.2737 | 4.1865 |
| Lungs | 4.3289 | 4.2032 | 4.4283 | 3.9506 |
| Liver | 1.2396 | 1.3142 | 1.3462 | 1.2306 |
| Spleen | 1.8158 | 1.8232 | 1.5867 | 2.0015 |
| Kidneys | 1.0857 | 1.0329 | 0.8553 | 1.0148 |
| Adrenal body | 5.9823 | 4.7996 | 7.5435 | 5.0644 |
| Thyroid gland | 0.1372 | 0.1414 | 0.1306 | 0.1725 |

**Table 10**

| % Dose/g | | | | |
|---|---|---|---|---|
| | α-Thioglycerol | Cysteine hydrochloride | Sodium thioglycollate | Control |
| Blood | 0.1804 | 0.1482 | 0.1577 | 0.1827 |
| Heart | 2.4007 | 2.0792 | 2.3911 | 2.2407 |
| Lungs | 1.1633 | 0.9324 | 1.1000 | 1.1303 |
| Liver | 0.3241 | 0.3073 | 0.3821 | 0.3434 |
| Spleen | 1.8158 | 1.8232 | 1.5867 | 2.0015 |
| Kidneys | 0.5286 | 0.4931 | 0.4528 | 0.3987 |
| Adrenal body | 3.1804 | 2.8411 | 2.7372 | 1.9155 |
| Thyroid gland | 0.2797 | 0.3080 | 0.2975 | 0.3701 |

### Reference Example 2

### Labeling of 15-(4-iodophenyl)-9-methylpentadecanoic acid (9MPA) labeled with radioactive iodine 123

1 g of 9MPA (manufactured by Fuji Pharmaceutical Co., Ltd.) was added to a 50 ml eggplant-type flask and dissolved in 4 ml of ethanol (manufactured by Kishida Chemical Co., Ltd.). Then, 150 µl of 20% acetic acid solution prepared by mixing glacial acetic acid (manufactured by Kishida Chemical Co., Ltd.) and distilled water in a ratio of 1:4, and 40 µl of an aqueous solution of copper sulfate prepared using water for injection at a concentration of 5mg/ml (manufactured by Kishida Chemical Co., Ltd.) were added.

Next, 200 µl of 0.1 M sodium hydroxide solution which contains radioactive iodine 123 in the form of sodium iodide with a radioactivity capacity of 25 GBq per 1 ml (manufactured by Daiichi Radioisotope Laboratories, Ltd.) was added. This eggplant-type flask was embedded in a heating block which was heated at 160-165°C. The solution was stirred using the stirrer for 10 minutes while slightly reducing the pressure using a pressure reducing pump, thereby carrying out both the reaction and concentration to dryness. After stopping the stirrer, the reaction was continued for a further 20 minutes under the same conditions. After the reaction, the reaction product was allowed to cool at room temperature for 5 minutes, and then dissolved in 4 ml of ethanol.

An anion exchange resin (Muromack 1-X8, 200-400 mesh, C1 Form, manufactured by Muromachi Chemical Co., Ltd.) sufficiently swelled in ethanol was filled in a 5 ml Thermo syringe up to 3 ml. All the reaction solution in ethanol was added to this column and collected in the same 50 ml eggplant-type flask as that used for the reaction. In addition, the eggplant-type flask which was used for the reaction was washed with 4 ml of ethanol and the washings were also added to the above column. Furthermore, 3 ml of ethanol was added to the column as the final elusion. This eggplant-type flask in which the reaction solution was collected from the column was embedded in a heating block which was heated at 140-145°C, while adding nitrogen gas to the eggplant-type flask at a rate of 200 ml/min. The solution was concentrated to dryness while slightly reducing the pressure using a pressure reducing pump for 15 minutes.

Upon confirming that the reaction product was concentrated to dryness, a solution of 2-hydroxypropyl-β-cyclodextrin (hereinafter called HP-β-CyD) adjusted to a concentration of 20 mg/ml using a 20 mM Tris HCl buffer (pH 8.0) containing 0.9% sodium chloride was added so that the radioactivity became 80 MBq/ml at noon of the following day. The reaction vessel was embedded in a heating block at 80°C and encapsulated for 30 minutes while stirring. After the encapsulation, the product was filtered using a 0.2 µm filter (Dimex 13, manufactured by Millipore Co.) to obtain the final preparation of radioactive iodine 123-labeled 9MPA.

10 ml of the radioactive iodine 123-labeled 9MPA solution thus obtained was dispensed into a 50 ml eggplant-type flask. The flask was capped with a rubber stopper equipped with a glass tube having a 0.2 µm filter and a glass ball filter for introducing helium gas at the top thereof. Helium gas was fed for 10 minutes at a flow rate of 150 ml/min.

After the helium gas charge, the rubber stopper was replaced by a ground stopper equipped with a glass tube connected to a vacuum controller via a cock. The internal pressure of the flask was instantly reduced to 45 Torr using a vacuum pump and the vacuum controller, then gradually reduced to 22 Torr, at which point the flask was allowed to stand for 5 minutes. After the pressure was restored to atmospheric, nitrogen gas was fed into the flask to fill the flask with nitrogen gas.

### Example 11

### Addition of α-thioglycerol (1-thioglycerol) to a solution of radioactive iodine 123-labeled 15-(4-iodophenyl)-9-methylpentadecanoic acid (2)

1 ml of the radioactive iodine 123-labeled 9MPA solution prepared in Reference Example 2 was added to a 5 ml glass bottle under a nitrogen stream. To 1 ml of the resulting solution, 5 µl (6.13 mg), 3 µl (3.68 mg), or 1 µl (1.23 mg) of 98% α-thioglycerol (manufactured by Sigma Co.) was added under a nitrogen stream. The mixture was stirred sufficiently. After stirring, the bottle was capped with a rubber stopper, thereby obtaining the radioactive medicine. In this instance, the glass bottle head space was saturated with nitrogen gas.

### Example 12

### Measurement of radiochemical purity

The decrease of radiochemical purity in the radioactive medicine of the present invention prepared in Example 11 was measured in the same manner as in Example 9, after the medicine was stored for 24 hours or 48 hours at 25°C or 37°C.

The same reverse phase thin layer chromatography as used in Example 9 was used for the measurement. A developing solution made by homogeneously mixing tetrahydrofuran (Kishida Chemical Co., Ltd., special quality reagent), acetonitrile (Kishida Chemical Co., Ltd., special quality reagent), distilled water, and acetic acid (Kishida Chemical Co., Ltd., special quality reagent) in a ratio of 40:40:20:1 was used.

The transfer rate of radioactive iodine 123 alone in this reverse phase thin layer chromatography was about 0.8-0.9, whereas the transfer rate of the radioactive iodine 123-labeled 9MPA was 0.2, indicating that it is possible to quantitatively measure the radioactive iodine 123 which is present unreacted in the solution or released from 9MPA.

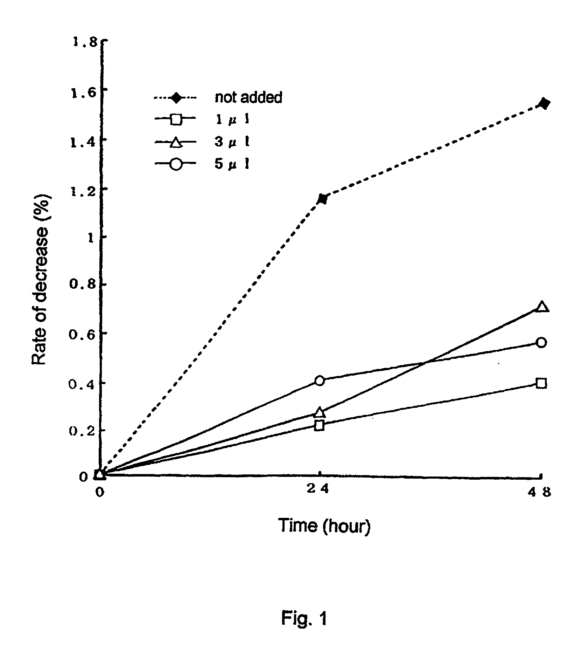

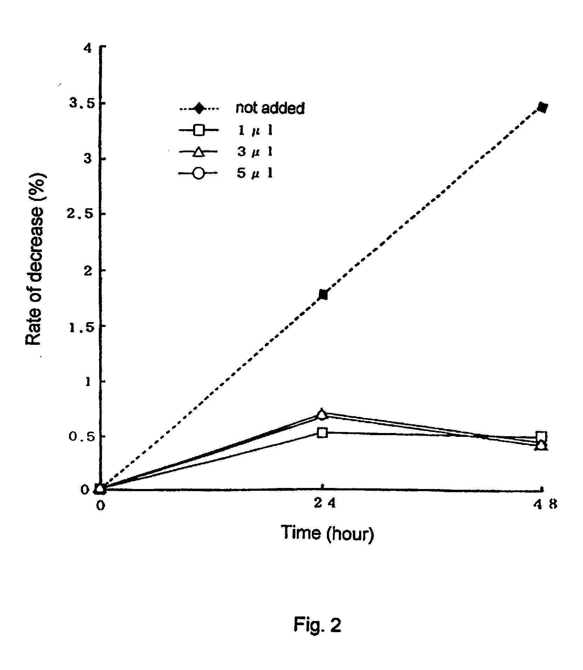

The results obtained by storing the sample at 25°C and 37°C are shown respectively in Fig. 1 and Fig. 2.

The radioactive medicine of the present invention to which α-thioglycerol was added was thus proven to be stable and exhibited only a slight decrease in the purity when stored at 25°C and 37°C.

As a result of the above test, the deiodination inhibitor used in the radioactive medicine of the present invention was confirmed to have no effect on the accumulation of radioactive iodine-labeled compounds in the heart, blood, and other organs, and the radioactive medicine of the present invention was confirmed to exhibit the same accumulation as the control product.

### INDUSTRIAL APPLICABILITY

Because the radioactive medicine of the present invention exhibits a controlled deiodination reaction and is stable, the medicine has the following excellent effect and is extremely useful.
(1) The radiochemical purity of radioactive medicines which were labeled with radioactive iodine can be maintained stable for a long period of time by the addition of a deiodination inhibitor.
(2) Because previous administration of non-radioactive iodine preparation can be eliminated, the medicine is extremely effective in accelerating examinations in hospitals.
(3) Because the radioactive medicine of the present invention reduces accumulation of radioactive iodine in the thyroid gland, the medicine is extremely effective in preventing unnecessary exposure of the thyroid gland to radiation.
(4) Because the radioactive medicine can be transported at an ambient temperature without the need for refrigeration for the prevention of deiodination, transportation costs can be reduced.
(5) Because cooling while storage and transportation is unnecessary, the procedure of warming the medicine to room temperature before administration can be eliminated. In addition, an unnecessary pain to the subjects due to administration of a cold medicine can be removed.
(6) Dissipation of radioactive iodine can be suppressed by securing a high radiochemical purity immediately after preparation. Contamination of the manufacturing plant can be prevented in the manufacture of conventional injection capsule preparations.

## Claims

1. A stable radioactive medicine comprising a radioactive iodine labeled compound and a deiodination inhibitor which is selected from the group consisting of α-thioglycerol, sodium hydrogensulfite, sodium pyrosulfite, cysteine hydrochloride, sodium edetate, sodium thioglycollate, and butylhydroxyanisole.

2. The stable radioactive medicine according to claim 1, wherein the deiodination inhibitor is α-thioglycerol which is contained in an amount from 0.5 to 25 mg/ml in the radioactive medicine.

3. The stable radioactive medicine according to claim 1, wherein the deiodination inhibitor is sodium hydrogensulfite which is contained in an amount from 5.2 to 800 mg/ml in the radioactive medicine.

4. The stable radioactive medicine according to claim 1, wherein the deiodination inhibitor is sodium pyrosulfite which is contained in an amount from 5.7 to 40 mg/ml in the radioactive medicine.

5. The stable radioactive medicine according to claim 1, wherein the deiodination inhibitor is cysteine hydrochloride which is contained in an amount from 3.5 to 25 mg/ml in the radioactive medicine.

6. The stable radioactive medicine according to claim 1, wherein the deiodination inhibitor is sodium edetate which is contained in an amount from 9.3 to 16 mg/ml in the radioactive medicine.

7. The stable radioactive medicine according to claim 1, wherein the deiodination inhibitor is sodium thioglycollate which is contained in an amount from 17 to 20 mg/ml in the radioactive medicine.

8. The stable radioactive medicine according to claim 1, wherein the deiodination inhibitor is butylhydroxyanisole which is contained in an amount from 22.5 to 75 µg/ml in the radioactive medicine.

9. The stable radioactive medicine according to claim 1, wherein the radioactive iodine-labeled compound is radioactive iodine 123-labeled 3-iodo benzyl guanidine, radioactive iodine 131-labeled 3-iodo benzyl guanidine, radioactive iodine 123-labeled 15-(4-iodophenyl)-9-methylpentadecanoic acid, or radioactive iodine 123-labeled N-isopropyl-p-iodo amphetamine.

10. A method for stabilizing a medicine containing radioactive iodine labeled compound, which comprises adding a deiodination inhibitor selected from the group consisting of α-thioglycerol, sodium hydrogensulfite, sodium pyrosulfite, cysteinehydrochloride, sodium edetate, sodium thioglycollate, and butylhydroxyanisole to the medicine containing radioactive iodine labeled compound.

## Patentansprüche

1. Stabile radioaktive Medizin, umfassend eine mit radioaktivem Iod markierte Verbindung und einen Deiodierungsinhibitor, der ausgewählt ist aus der Gruppe, bestehend aus α-Thioglycerin, Natriumhydrogensulfit, Natriumpyrosulfit, Cysteinhydrochlorid, Natriumedetat, Natriumthioglykolat und Butylhydroxyanisol.

2. Stabile radioaktive Medizin gemäss Anspruch 1, wobei der Deiodierungsinhibitor α-Thioglycerin ist, das in einer Menge von 0,5 bis 25 mg/ml in der radioaktiven Medizin enthalten ist.

3. Stabile radioaktive Medizin gemäss Anspruch 1, wobei der Deiodierungsinhibitor Natriumhydrogensulfit ist, das in einer Menge von 5,2 bis 800 mg/ml in der radioaktiven Medizin enthalten ist.

4. Stabile radioaktive Medizin gemäss Anspruch 1, wobei der Deiodierungsinhibitor Natriumpyrosulfit ist, das in einer Menge von 5,7 bis 40 mg/ml in der radioaktiven Medizin enthalten ist.

5. Stabile radioaktive Medizin gemäss Anspruch 1, wobei der Deiodierungsinhibitor Cysteinhydrochlorid ist, das in einer Menge von 3,5 bis 25 mg/ml in der radioaktiven Medizin enthalten ist.

6. Stabile radioaktive Medizin gemäss Anspruch 1, wobei der Deiodierungsinhibitor Natriumedetat ist, das in einer Menge von 9,3 bis 16 mg/ml in der radioaktiven Medizin enthalten ist.

7. Stabile radioaktive Medizin gemäss Anspruch 1, wobei der Deiodierungsinhibitor Natriumthioglykolat ist, das in einer Menge von 17 bis 20 mg/ml in der radioaktiven Medizin enthalten ist.

8. Stabile radioaktive Medizin gemäss Anspruch 1, wobei der Deiodierungsinhibitor Butylhydroxyanisol ist, das in einer Menge von 22,5 bis 75 µg/ml in der radioaktiven Medizin enthalten ist.

9. Stabile radioaktive Medizin gemäss Anspruch 1, wobei die mit radioaktivem Iod markierte Verbindung ein mit radioaktivem Iod 123 markiertes 3-Iodbenzylguanidin, mit radioaktivem Iod 131 markiertes 3-Iodbenzylguanidin, mit radioaktivem Iod 123 markierte 15-(4-Iodphenyl)-9-methylpentadecansäure oder mit radioaktivem Iod 123 markiertes N-Isopropylp-iodamphetamin ist.

10. Verfahren zur Stabilisierung einer Medizin, enthaltend eine mit radioaktivem Iod markierte Verbindung, das die Zugabe eines Deiodierungsinhibitors, ausgewählt aus der Gruppe, bestehend aus α-Thioglycerin, Natriumhydrogensulfit, Natriumpyrosulfit, Cysteinhydrochlorid, Natriumedetat, Natriumthioglykolat und Butylhydroxyanisol, zu der Medizin, die die mit radioaktivem Iod markierte Verbindung enthält, umfasst.

## Revendications

1. Médicament radioactif stable comprenant un composé marqué par de l'iode radioactif et un inhibiteur de désiodation qui est choisi dans le groupe constitué par l'α-thioglycérol, l'hydrogénosulfite de sodium, le pyrosulfite de sodium, le chlorhydrate de cystéine, l'édétate de sodium, le thioglycollate de sodium et le butylhydroxyanisole.

2. Médicament radioactif stable selon la revendication 1, dans lequel l'inhibiteur de désiodation est l'α-thioglycérol qui est contenu en une quantité de 0,5 à 25 mg/ml dans le médicament radioactif.

3. Médicament radioactif stable selon la revendication 1, dans lequel l'inhibiteur de désiodation est l'hydrogénosulfite de sodium qui est contenu en une quantité de 5,2 à 800 mg/ml dans le médicament radioactif.

4. Médicament radioactif stable selon la revendication 1, dans lequel l'inhibiteur de désiodation est le pyrosulfite de sodium qui est contenu en une quantité de 5,7 à 40 mg/ml dans le médicament radioactif.

5. Médicament radioactif stable selon la revendication 1, dans lequel l'inhibiteur de désiodation est le chlorhydrate de cystéine qui est contenu en une quantité de 3,5 à 25 mg/ml dans le médicament radioactif.

6. Médicament radioactif stable selon la revendication 1, dans lequel l'inhibiteur de désiodation est l'édétate de sodium qui est contenu en une quantité de 9,3 à 16 mg/ml dans le médicament radioactif.

7. Médicament radioactif stable selon la revendication 1, dans lequel l'inhibiteur de désiodation est le thioglycollate de sodium qui est contenu en une quantité de 17 à 20 mg/ml dans le médicament radioactif.

8. Médicament radioactif stable selon la revendication 1, dans lequel l'inhibiteur de désiodation est le butylhydroxyanisole qui est contenu en une quantité de 22,5 à 75 µg/ml dans le médicament radioactif.

9. Médicament radioactif stable selon la revendication 1, dans lequel le composé marqué par de l'iode radioactif est la 3-iodobenzylguanidine marquée par de l'iode 123 radioactif, la 3-iodobenzylguanidine marquée par de l'iode 131 radioactif, l'acide 15-(4-iodophényl)-9-méthylpentadécanoïque marqué par de l'iode 123 radioactif ou la N-isopropyl-p-iodoamphétamine marquée par de l'iode 123 radioactif.

10. Procédé de stabilisation d'un médicament contenant un composé marqué par de l'iode radioactif, qui comprend l'addition d'un inhibiteur de désiodation choisi dans le groupe constitué par l'α-thioglycérol, l'hydrogénosulfite de sodium, le pyrosulfite de sodium, le chlorhydrate de cystéine, l'édétate de sodium, le thioglycollate de sodium et le butylhydroxyanisole, au médicament contenant le composé marqué par de l'iode radioactif.
